# EUROPÄISCHE PATENTSCHRIFT

(11) **EP 1 988 019 B1**
(45) Veröffentlichungstag und Bekanntmachung des Hinweises auf die Patenterteilung: **21.01.2009**
(21) Anmeldenummer: 07008966.9
(22) Anmeldetag: 03.05.2007
(51) Int. Cl.: B65B 5/10, B65B 11/50, A61J 7/00

(54) **Verfahren zur Herstellung von Mehrtagesverpackungen mit verschiedenen Arzneimitteln**
Method for manufacturing supply packs with various medicines for several days
Procédé de fabrication d'emballage pour plusieurs jours de divers médicaments

(43) Veröffentlichungstag der Anmeldung: 05.11.2008
(62) Teilanmeldung aus: 07024522.0
(73) Patentinhaber: UHLMANN PAC-SYSTEME GmbH & Co. KG, 88471 Laupheim (DE)
(72) Erfinder: Drost, Siegfried, 88471 Laupheim (DE); Felk, Günter, 89079 Ulm (DE); Pfau, Georg, 88471 Laupheim (DE); Bongers-Ambrosius, Hans-Werner, 88471 Laupheim (DE); Prinz, Heino, Dr., 88471 Laupheim (DE); Mertens, Richard, 88471 Laupheim (DE)
(74) Vertreter: Wächter, Jochen

(56) Entgegenhaltungen:
- WO-A-20/05075293
- DE-A1- 19 926 893
- DE-A1-0102004 020 51
- DE-A1-4102004 034 02
- DE-A1-9102005 047 42
- DE-B3-2102005 049 88

## Beschreibung

Die Erfindung betrifft ein Verfahren zur Herstellung von Mehrtagesverpackungen, die für mehrere Tage und mehrere Einnahmezeiten verschiedene Arzneimittel beinhalten.

Herkömmliche Blisterverpackungen sind auf die Aufnahme eines einzigen Medikaments in mehreren Blisterhöfen ausgerichtet. Eine Vorrichtung zur Befüllung derartiger herkömmlicher Blisterverpackungen ist beispielsweise aus der DE 199 26 893 C2 bekannt.

Bei der Behandlung chronisch kranker Menschen lässt jedoch oftmals die konsequente, therapietreue Einnahme von Medikamenten zu wünschen übrig. Dies ist insbesondere dann der Fall, wenn mehrmals täglich verschiedene Tabletten eingenommen werden müssen. Um einerseits eine Erinnerungshilfe zur Einnahme der Medikamente zu schaffen und andererseits einen zuverlässigen Überblick zu gewähren, welche Medikamente bereits eingenommen sind, ist in der DE 10 2004 034 024 A1 eine Blisterverpackung beschrieben, in der die Arzneimittelhöfe in einer nach Wochentagen und Einnahmezeiten unterteilten Matrix mit sieben Zeilen und mindestens drei Spalten angeordnet sind. Die einzelnen Arzneimittelhöfe sind dabei als wannenförmige Vertiefungen einer dünnen Kunststoffschicht ausgebildet, und alle Tabletten, die für einen Einnahmezeitpunkt vorgesehen sind, sind gemeinsam in einer solchen Vertiefung aufgenommen. Alle Arzneimittelhöfe sind mit einer Blisterfolie abgedeckt und die Abschnitte für die einzelnen Einnahmezeitpunkte sind jeweils mit Perforationen voneinander getrennt, so dass eine einfache Separierung einzelner Einnahmeabschnitte für bestimmte Tageszeiten und bestimmte Wochentage möglich ist.

Ein Verfahren zur Herstellung einer derartigen Verpackungseinheit für den wöchentlichen Medikamentenbedarf eines Patienten ist beispielsweise aus DE 10 2004 020 510 A1 bekannt. Hierbei werden streifenförmige, zu Rollen aufgewickelte Vorratsblisterverpackungen, welche die einzelnen Medikamente beinhalten, an verschiedenen Ausgabestationen über der oben beschriebenen Verpackungseinheit in Querrichtung bewegt und mittels Stößeln aus der streifenförmigen Vorratsblisterverpackung in die wannenförmigen Vertiefungen der Verpackungseinheit gedrückt, bevor die als Wochenblister ausgebildete Verpackungseinheit integral mit einer Versiegelungsfolie versiegelt wird.

Das oben erwähnte Herstellungsverfahren ist aufgrund der komplizierten Mechanik und des doppelt vorhandenen Verpackungsschritts der Tabletten jedoch relativ umständlich und auch aus Kontaminationsgesichtspunkten kritisch, da beim Ausdrückvorgang der Tabletten mittels der Stößel eine Menge an Tablettenstaub erzeugt wird, der sich an den Stößeln und in den wannenförmigen Vertiefungen ablagert.

Ein weiteres Herstellungsverfahren ist aus der DE 10 2005 047 429 A1 bekannt. Darin ist eine Anlage zur Bestückung von Wochenblistern offenbart, die in erster Linie für den Fall gedacht ist, dass die Zahl der Patienten vergleichsweise gering ist (z.B. in einem Klinikum). Es werden handelsübliche Blisterverpackungen gestapelt in Sektormagazinen aufbewahrt, die jeweils gedreht werden können, um einen Blister der gewünschten Tablettenart freizugeben. Der Blisterhof, aus dem die Tablette entnommen werden soll, wird dann zunächst durch Ausstanzen der Blisterkappe geöffnet. Die Blisterkappe wird anschließend durch eine in das Stanzwerkzeug integrierte Saugpipette entfernt, woraufhin die Tablette ebenfalls mittels der Saugpipette aus dem Blisterhof entnommen und in den Wochenblister gefüllt wird. Das gesamte Verfahren ist äußerst aufwändig.

Der vorliegenden Erfindung liegt daher die Aufgabe zugrunde, ein Herstellungsverfahren für eine Mehrtagesverpackung mit verschiedenen Arzneimitteln zu schaffen, das eine Kontamination ausschließt und auf einfache Weise und zudem sehr schnell durchgeführt werden kann. Diese Aufgabe wird durch die Merkmale des Anspruchs 1 gelöst.

Erfindungsgemäß weist das Verfahren zur Herstellung einer Mehrtagesverpackung mit verschiedenen Arzneimitteln folgende Schritte auf: Formen einer Bodenschicht mit einer Vielzahl von in einem Muster angeordneten Vertiefungen; Anordnen von Vorratsblisterbahnen mit einer vorbestimmten Anzahl von jeweils verschiedenen Arzneimitteln an vorbestimmten Positionen an verschiedenen Ausgabestationen einer Füllvorrichtung, wobei die Vorratsblisterhöfe mittels einer vorläufigen Verriegelungsfolie, die auf Stegen der Folienbahn zwischen den Vorratsblisterhöfen haftend angebracht wird, verschlossen sind; teilweises Abläsen der vorläufigen Verriegelungsfolie zur Freigabe von Arzneimitteln; Aufnehmen einer gewünschten Anzahl an Arzneimitteln aus den vorbestimmten Positionen mittels mindestens einer beweglichen Ansaug- oder Greifvorrichtung; Ablegen der Arzneimittel in vorbestimmten Vertiefungen; und Aufbringen einer Versiegelungsfolie auf die Bodenschicht zum Verschluss der Vertiefungen.

So wird eine Mehrtagesverpackung mit verschiedenen Arzneimitteln auf besonders einfache Weise, schnell und ohne Kontaminationsgefahr hergestellt, wobei die Arzneimittel auch bei längerer Lagerung gasdicht in den Vorratsblisterhöfen aufbewahrt werden.

Vorzugsweise weist die Herstellung der Vorratsblisterbahn folgende Schritte auf: Formen einer sich in Längs- und Querrichtung weit erstreckenden Bodenbahn, wodurch die Vielzahl von Vorratsblisterhöfen entsteht; und Zuführen von Arzneimitteln in die Vorratsblisterhöfe.

Dabei kann es vorteilhaft sein, vor dem Schritt des Ablösens der vorläufigen Versiegelungsfolie von der Vorratsblisterbahn die Vorratsblisterbahn zu Vorratsblisterabschnitten zu schneiden und die Vorratsblisterabschnitte an der Ausgabestation quer zur Transportrichtung der Mehrtagesverpackung auf einer Rolle anzuordnen. Hierdurch wird ein gezielter Zugriff auf einzelne, selten verwendete Tabletten möglich. Die Vorratsblisterabschnitte sind nicht notwendigerweise auf eine einzige streifenförmige Reihe von Vorratsblisterhöfen beschränkt, sondern können auch mehrere Vorratsblisterhöfe nebeneinander aufweisen, so dass eine gleichzeitige Entnahme von Arzneimitteln aus mehreren Vorratsblisterhöfen durch mehrere Ansaug- oder Greifvorrichtungen nicht nur quer, sondern auch längs zur Transportrichtung der Mehrtagesverpackung möglich ist.

Zur einfachen Qualitätskontrolle wird die Ansaug- oder Greifvorrichtung vorzugsweise mittels einer Kamera oder durch Sensoren überwacht.

Durch den weiteren Schritt, in die Bodenschicht und die Versiegelungsfolie zwischen den Vertiefungen Perforationen einzubringen, erhält man die Möglichkeit, einzelne Abschnitte für bestimmte Einnahmezeiten oder bestimmte Tage von der Mehrtagesverpackung abzutrennen.

Weitere Einzelheiten, Merkmale und Vorteile der vorliegenden Erfindung ergeben sich aus der nachfolgenden Beschreibung unter Bezugnahme auf die Zeichnungen.
- Fig. 1: ist eine schematische Draufsicht auf eine Mehrtagesverpackung mit verschiedenen Arzneimitteln bei teilweise entfernter Versiegelungsfolie;
- Fig. 2: ist eine Querschnittsansicht der Mehrtagesverpackung aus Fig. 1;
- Fig. 3: ist ein Ablaufdiagramm des erfindungsgemäßen Verfahrens zur Herstellung der Mehrtagesverpackung;
- Fig. 4: ist ein Ablaufdiagramm einer Alternative zum erfindungsgemäßen Verfahren zur Herstellung der Mehrtagesverpackung;
- Fig. 5: ist eine schematische Ansicht einer Füllvorrichtung, die beim erfindungsgemäßen Herstellungsverfahren verwendet werden kann; und
- Fig. 6: ist eine schematische Ansicht einer Füllvorrichtung, die beim alternativen Herstellungsverfahren verwendet werden kann.

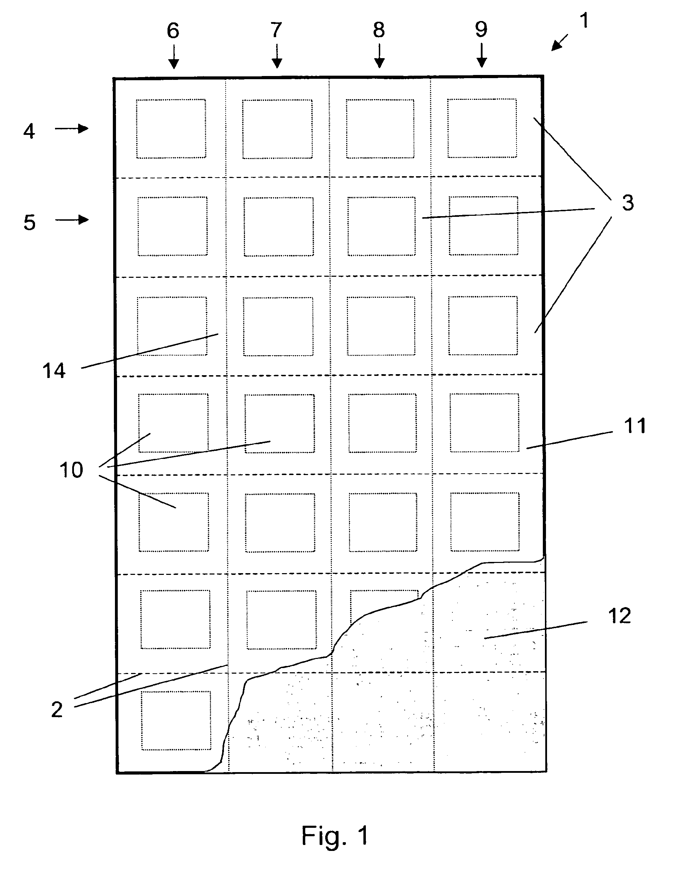

Fig. 1 zeigt eine schematische Draufsicht auf ein Beispiel einer Mehrtagesverpackung 1. Die Verpackungseinheit 1 umfasst mehrere Segmente 3, von denen jedes einer bestimmten Tageszeit eines bestimmten Wochentags zugeordnet ist und mehrere Medikamente aufnehmen kann. Die Segmente 3 sind in Zeilen und Spalten angeordnet und vorzugsweise mittels Perforationen 2 getrennt, wobei im dargestellten Ausführungsbeispiel die erste Zeile 4 beispielsweise für den Wochentag "Montag" steht, die zweite Zeile 5 für den Wochentag "Dienstag", usw. Die Einteilung in Spalten entspricht den unterschiedlichen Einnahmezeiten für Medikamente während eines bestimmten Wochentages, wobei beispielsweise die erste Spalte 6 der Einnahmezeit "Morgen" zugeordnet ist, die zweite Spalte 7 der Einnahmezeit "Mittag", die dritte Spalte 8 der Einnahmezeit "Abend" und die vierte Spalte 9 der Einnahmezeit "Nacht". Es ist auch möglich, Arzneimittel für mehr oder weniger als sieben Tage (z.B. Monatsblister) oder auch auf bestimmte Einnahmezeitpunkte beschränkt in die Mehrtagesverpackung zu integrieren.

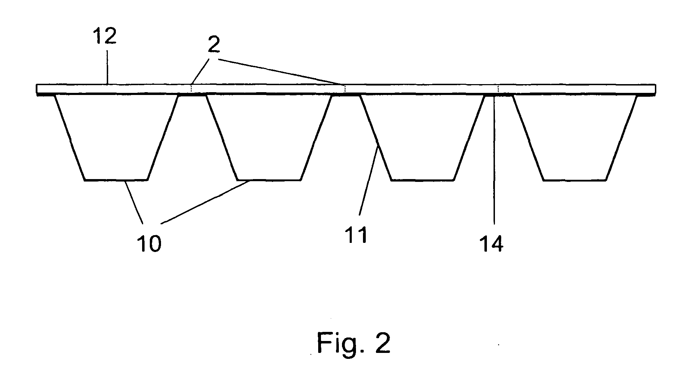

In Fig. 2 ist ein Ausschnitt einer Verpackungseinheit 1 mit mehreren nebeneinander angeordneten Segmenten 3 in Querschnittsansicht dargestellt. Die Segmente 3 sind aus einer Bodenschicht 11 gebildet, in die jeweils eine relativ großflächige Vertiefung 10 zur Aufnahme mehrerer Arzneimittel geformt ist. Die Bodenschicht 11 besteht vorzugsweise aus transparenter oder beliebig gefärbter PVC-Folie, aus Polypropylen oder aus einer Aluminiumverbundschicht. Die Vertiefungen 10 können auch eine beliebige andere als die in Fig. 2 dargestellte wannenförmige Gestalt aufweisen und können auch in nahezu beliebigen regelmäßigen oder unregelmäßigen Mustern, z.B. kreisförmig, angeordnet sein.

Jede Vertiefung 10 ist mit einer Versiegelungsfolie 12 verschlossen, die mit den zwischen den Vertiefungen 10 angeordneten Stegen 14 der Bodenschicht 11 haftend verbunden ist. Als Versiegelungsfolie 12 kommen beispielsweise Aluminiumfolien, papierkaschierte Folien oder Polypropylenfolien in Betracht. Die Versiegelungsfolie 12 weist im Überdeckungsbereich der Vertiefung 10 keine Perforation auf, sodass ein sicherer und dauerhafter Verschluss der Arzneimittel bis zum Gebrauch gewährleistet ist. In den Stegen 14 der Bodenschicht 11, die die Vertiefungen 10 begrenzen, und der Versiegelungsfolie 12 sind die Perforationen 2 zwischen den einzelnen Segmenten 3 zu erkennen.

Bei einem Abziehen der Versiegelungsfolie 12 von den Stegen 14 um eine Vertiefung 10 kann somit auf sämtliche Medikamente, die für einen Einnahmezeitpunkt vorgesehen sind, auf einmal zugegriffen werden. Hierzu ist wichtig, dass die Versiegelungsfolie 12 relativ reißfest ausgebildet ist.

Die Versiegelungsfolie 12 kann zusätzlich mit einer Auflistung der jeweils in den Vertiefungen 10 enthaltenen Medikamente beschriftet sein. Ebenso ist es denkbar, einen zusätzlichen Klappdeckel vorzusehen, auf den Angaben zu den Medikamenten aufgedruckt sind.

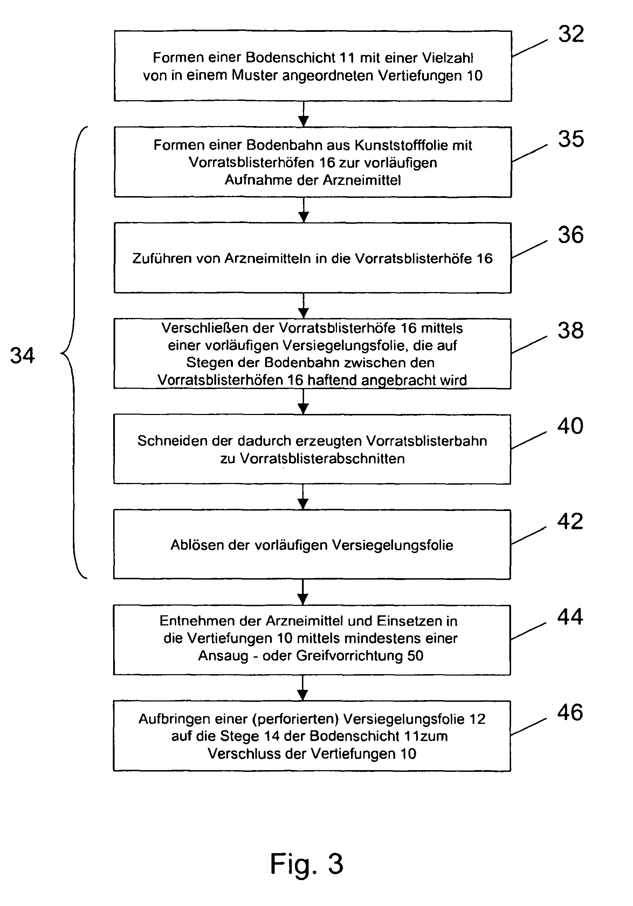

Ein Ablaufdiagramm des erfindungsgemäßen Verfahrens zum Herstellen der Mehrtagesverpackung ist in Fig. 3 dargestellt.

Zunächst werden in eine Bodenschicht 11 Vertiefungen 10 zur Aufnahme der Arzneimittel geformt (Schritt 32). Hierzu wird die Bodenschicht 11 von einer Rolle abgespult, gegebenenfalls in einer Heizstation geheizt und in einer anschließenden Formstation mit den Vertiefungen 10 versehen.

Anschließend erfolgt das Anordnen (Schritt 34) einer vorbestimmten Anzahl von jeweils verschiedenen Arzneimitteln in vorbestimmten Positionen an verschiedenen Ausgabestationen der Füllstation. Die bei der Ausführungsform von Fig. 3 hierfür durchzuführenden Schritte werden nachfolgend erläutert.

Zunächst wird in Schritt 35 eine sich in Längs- und Querrichtung weit erstreckende Bodenbahn geformt, wodurch eine Vielzahl von Vorratsblisterhöfen 16 entsteht. Hierzu wird die Bodenbahn von einer Rolle abgewickelt, gegebenenfalls in einer Heizstation erhitzt und in einer Formstation mit den Vorratsblisterhöfen 16 versehen. Anschließend erfolgt das Befüllen der Vorratsblisterhöfe 16 mit Arzneimitteln (Schritt 36) sowie vorzugsweise eine Füllkontrolle mit angekoppeltem Produktauswurf. Im nachfolgenden Schritt 38 werden die Vorratsblisterhöfe 16 mittels einer vorläufigen Versiegelungsfolie, die auf Stegen der Bodenbahn zwischen den Vorratsblisterhöfen 16 haftend angebracht wird, verschlossen. Hierzu wird die vorläufige Versiegelungsfolie von einer Rolle abgespult, in einer Siegelstation auf die Stege der Bodenbahn zwischen den Vorratsblisterhöfen 16 aufgebracht und anschlie-ßend in einer Kühlstation abgekühlt.

Anschließend wird die so erzeugte großflächige Vorratsblisterbahn in einzelne Vorratsblisterabschnitte geschnitten (Schritt 40). Das Schneiden der Vorratsblisterbahn in separierte Vorratsblisterabschnitte umfasst vorzugsweise das Schneiden der Verpackungsbahn in Längsrichtung mittels eines Längsschneiders. Im Längsschneider wird die Vorratsblisterbahn in der Laufrichtung der Vorratsblisterbahn geschnitten. Der Schnitt erfolgt vorzugsweise jeweils mit einem Rundmesser auf einer Welle. Diese Welle befindet sich in der Regel unterhalb der Vorratsblisterbahn. Oberhalb der Vorratsblisterbahn befindet sich eine Welle mit je einer Rundscheibe pro Rundmesser. Die Schnittfläche wird zwischen Rundmessern und der Rundscheibe gebildet. Niederhalter drücken die Vorratsblisterbahn von oben gegen die Führung, um einen geraden Schnitt zu erreichen. Der Längsschneider wird üblicherweise über einen Zahnriemen von einem Motor angetrieben.

Hierdurch werden Vorratsblisterabschnitte der Vorratsblisterbahn erzeugt, die anschließend zu Rollen aufgewickelt werden. Ein Vorratsblisterabschnitt kann streifenförmig sein, aber kann auch in Querrichtung mehrere Vorratsblisterhöfe 16 aufweisen. Nachfolgend werden die Vorratsblisterabschnitte von der Rolle abgewickelt und die vorläufige Versiegelungsfolie wird abgelöst (Schritt 42).

Anschließend werden die Medikamente gesteuert mittels mindestens einer beweglichen Ansaug- oder Greifvorrichtung 50 aufgenommen und in die Vertiefungen 10 eingesetzt (Schritt 44).

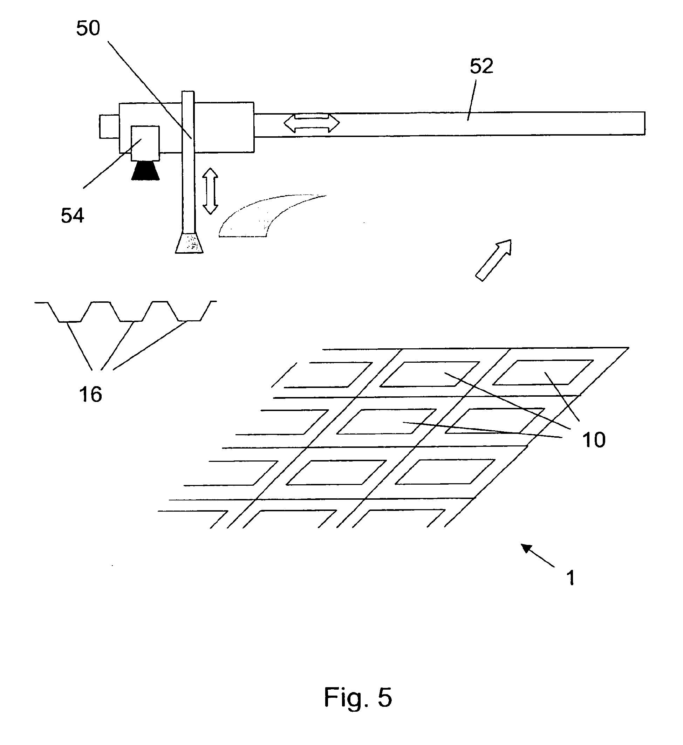

Ein Beispiel einer in Querrichtung beweglichen Ansaugvorrichtung 50 ist in Fig. 5 dargestellt, die eine schematische Ansicht einer Füllvorrichtung für das erfindungsgemäße Verfahren zeigt. In diesem schematisch dargestellten Ausführungsbeispiel ist die Ansaugvorrichtung 50 ein vertikal beweglicher Saugarm, der von einer Steuervorrichtung (nicht dargestellt) angesteuert wird und dazu vorgesehen ist, eine einzelne Tablette aus einem bereits von der Versiegelungsfolie 12 befreiten Vorratsblisterhof 16 aufzunehmen und in die jeweils gewünschte Vertiefung 10 in der Bodenschicht 11 einzufügen. Hierbei ist eine Bewegung in Querrichtung entlang einer Schiene 52 denkbar, aber auch jede andere geführte Bewegung, die von der Steuereinrichtung gesteuert werden kann, kann hier eingesetzt werden.

Es können auch mehrere Saugarme 50 parallel eingesetzt werden, die getrennt angesteuert werden, um zeitgleich mehrere Tabletten in die Vertiefungen 10 einzufügen. Der Transport der Bodenschicht 11 verläuft vorzugsweise getaktet, wobei die Befüllung der Mehrtagesverpackung 1 mit den einzelnen Tabletten zeilenmäßig erfolgt. Bei Vorliegen mehrerer Saugarme 50 kann auch eine gleichzeitige Befüllung mehrerer Zeilen bzw. Segmente 3 erfolgen. Hierzu kann es auch sinnvoll sein, wenn die Saugarme 50 auch entlang der Transportrichtung der Mehrtagesverpackung bewegbar sind.

Eine im Bereich der Ansaugvorrichtung 50 installierte und vorzugsweise mitbewegte Kamera 54 dient zur Überwachung sowohl des Ansaugvorgangs als auch zur Überwachung der korrekten Ablage der Tabletten in den Vertiefungen 10 der Bodenschicht 11. Auch andere Sensoren sind geeignet, um das Aufnehmen und Ablegen der Tabletten zu überwachen.

Als letzter Schritt erfolgt die Versiegelung der Mehrtagesverpackung mittels der Versiegelungsfolie 12 (Schritt 46).

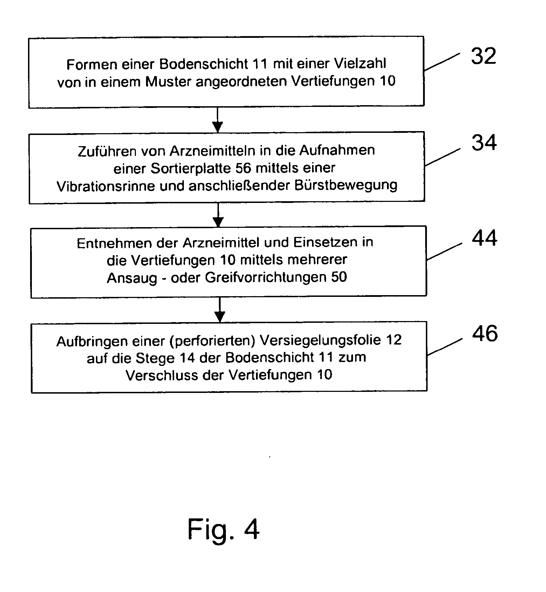

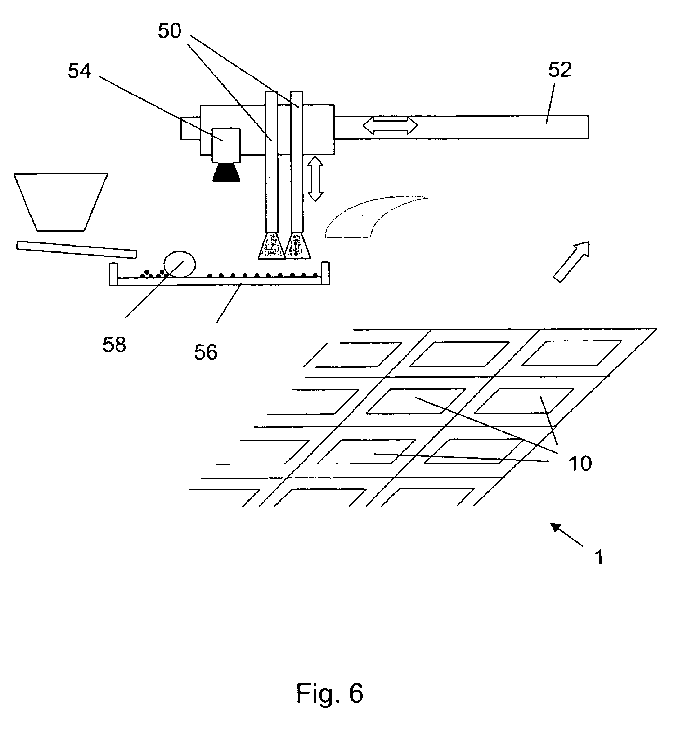

Zusätzlich zum erfindungsgemäßen Verfahren kann das Anordnen 34 einer vorbestimmten Anzahl eines Arzneimittels in vorbestimmten Positionen an anderen Ausgabestationen der Füllvorrichtung auch anders erfolgen, wie aus Fig. 4 und Fig. 6 ersichtlich ist.

Dabei werden entsprechend dem Muster der Vertiefungen 10 in der Mehrtagesverpackung angeordnete Aufnahmen einer Sortierplatte 56 mit Arzneimitteln befüllt. Diese Variante ist besonders sinnvoll, wenn an der Ausgabestation mehrere Ansaug- oder Greifvorrichtungen 50 einzeln angesteuert werden, die entsprechend dem Muster der Aufnahmen über der Sortierplatte 56 angeordnet werden können, und wenn eine große Anzahl dieser Arzneimittel in jeder Mehrtagesverpackung verpackt werden soll. Das Aufbringen der Arzneimittel auf die Sortierplatte 56 erfolgt vorzugsweise mittels einer Vibrationsrinne. Zur Sicherstellung der vollständigen Befüllung der Aufnahmen der Sortierplatte 56 erfolgt dann vorzugsweise noch eine Bürstbewegung mit einer Bürstvorrichtung 58, durch die auch überschüssige Tabletten von der Sortierplatte 56 entfernt werden.

Mit der in Fig. 6 schematisch dargestellten Füllvorrichtung, bei der aus Gründen der Übersichtlichkeit die Sortierplatte 56 verkleinert dargestellt ist und lediglich zwei Ansaugvorrichtungen 50 aus der dem Muster der Vertiefungen 10 der Mehrtagesverpackung entsprechenden Matrixanordnung der Ansaugvorrichtungen 50 dargestellt sind, kann somit ein beliebiges Muster von Arzneimitteln aus der Sortierplatte 56 entnommen und in die entsprechenden Vertiefungen 10 der Mehrtagesverpackung eingebracht werden, wobei die geleerten Aufnahmen anschließend wieder aufgefüllt werden.

Es ist möglich, die erfindungsgemäße und die alternative Ausführungsart des Verfahrens in Kombination, also an verschiedenen Ausgabestationen derselben Füllvorrichtung, anzuwenden.

Somit wird auf besonders einfache und schnelle Weise eine Mehrtagesverpackung für verschiedene Arzneimittel geschaffen, in der die Medikamente nach Einnahmezeiten sortiert vorliegen.

## Patentansprüche

1. Verfahren zur Herstellung einer Mehrtagesverpackung mit verschiedenen Arzneimitteln, mit folgenden Schritten:
- Formen (32) einer Bodenschicht (11) mit einer Vielzahl von in einem Muster angeordneten Vertiefungen (10);
- Anordnen (34) von Vorratsblisterbahnen an verschiedenen Ausgabestatione einer Füllvorrichtung, wobei die Vorratsblisterbahnen jeweils an vorbestimmten Positionen Vorratsblisterhöfe (16) zur Aufnahme einer Anzahl von an jeder Ausgabestation verschiedenen Arzneimitteln aufweisen, und wobei die Vorratsblisterhöfe (16) mittels einer vorläufigen Versiegelungsfolie, die auf Stegen der Bodenbahn zwischen den Vorratsbtisterhöfen (16) haftend angebracht wird, verschlossen sind (38);
- teilweises Ablösen (42) der vorläufigen Versiegelungsfolie von der Vorratsblisterbahn zur Freigabe einer vorbestimmten Anzahl an Arzneimitteln an die mindestens eine Ansaug- oder Greifvorrichtung (50);
- gesteuertes Aufnehmen (44) einer gewünschten Anzahl von Arzneimitteln aus den vorbestimmten Positionen an jeder Ausgabestation mittels mindestens einer beweglichen Ansaug- oder Greifvorrichtung (50) und Ablegen der Arzneimittel in vorbestimmten Vertiefungen (10); und
- Aufbringen (46) einer Versiegelungsfolie (12) auf die Bodenschicht (11) zum Verschluss der Vertiefungen (10).

2. Verfahren nach Anspruch 1, **dadurch gekennzeichnet, dass** die Herstellung der Vorratsblisterbahn folgende Schritte aufweiset:
- Formen (35) einer sich in Längs- und Querrichtung weit ersteckenden Bodenbahn, wodurch die Vielzahl von Vorratsblisterhöfen (16) entsteht; und
- Zuführen (36) von Arzneimitteln in die Vorratsblisterhöfe (16).

3. Verfahren nach Anspruch 2, **gekennzeichnet durch** den weiteren Schritt, vor dem Schritt des Ablösens (42) der vorläufigen Versiegelungsfolie von der Vorratsblisterbahn die Vorratsblisterbahn zu Vorratsblisterabschnitten zu schneiden und die Vorratsblisterabschnitte an der Ausgabestation quer zur Transportrichtung der Mehrtagesverpackung auf einer Rolle anzuordnen.

4. Verfahren nach einem der vorangehenden Ansprüche, **dadurch gekennzeichnet, dass** an mindestens einer Ausgabestation mehrere Ansaug- oder Greifvorrichtungen (50) einzeln angesteuert werden.

5. Verfahren nach Anspruch 4, **dadurch gekennzeichnet, dass** die Ansaug- oder Greifvorrichtungen (50) entsprechend dem Muster der Vertiefungen (10) in der Mehrtagesverpackung angeordnet sind und parallel zueinander quer zur Transportrichtung der Mehrtagesverpackung verfahren werden.

6. Verfahren nach einem der vorangehenden Ansprüche, **dadurch gekennzeichnet, dass** die mindestens eine Ansaug- oder Greifvorrichtung (50) jeweils mittels einer Kamera (54) oder durch Sensoren überwacht wird.

7. Verfahren nach einem der vorangehenden Ansprüche, **gekennzeichnet durch** die weiteren Schritte, in die Bodenschicht (11) und die Versiegelungsfolie (12) zwischen den Vertiefungen (10) Perforationen (2) einzubringen.

## Claims

1. A method of producing a multi-day package containing various drugs comprising the following steps:
-- forming (32) a bottom layer (11) with a plurality of wells (10) arranged in a pattern;
-- arranging (34) supply-blister sheets at various dispensing stations of a filling device, wherein, at predetermined positions at each dispensing station, the supply-blister sheets have supply-blister pockets (16) to hold a number of different drugs, and wherein the supply-blister pockets (16) are sealed by a temporary sealing film, which is applied (38) adhesively to the webs of the base layer between the supply-blister pockets (16);
-- partially removing (42) the temporary sealing film from the supply-blister sheet to release a predetermined number of drugs to at least one suction or gripper device (50);
-- picking (44) the desired drugs in a controlled manner from the predetermined positions at each dispensing station by at least one movable suction or gripper device (50), and placing the drugs in predetermined wells (10); and
-- applying (46) a sealing film (12) to the bottom layer (11) to seal off the wells (10).

2. The method according to claim 1, **characterized in that** the production of the supply-blister sheet comprises the following steps:
-- forming (35) a base layer extending a considerable distance in the longitudinal and transverse directions, as a result of which the plurality of supply-blister pockets (16) is obtained; and
-- loading (36) the drugs into the supply-blister pockets (16).

3. The method according to claim 2, **characterized by** the additional step that, before the step of removing (42) the temporary sealing film from the supply-blister sheet, the supply-blister sheet is cut into supply-blister sections, and the supply-blister sections are arranged on rolls at the dispensing station transversely to the transport direction of the multi-day package.

4. The method according to one of the preceding claims, **characterized in that** several suction or gripper devices (50) are actuated individually in at least one dispensing station.

5. The method according to claim 4, **characterized in that** the suction or gripper devices (50) are arranged in correspondence with the pattern of the wells (10) in the multi-day package and are moved parallel to each other transversely to the transport direction of the multi-day package.

6. The method according to one of the preceding claims, **characterized in that** the at least one suction or gripper device (50) is monitored by means of a camera (54) or by sensors.

7. The method according to one of the preceding claims, **characterized by** the additional steps that perforations (2) are introduced into the bottom layer (11) and into the sealing film (12) between the wells (10).

## Revendications

1. Procédé de fabrication d'un emballage pour plusieurs jours de médicaments différents, comprenant les étapes suivantes :
- formage (32) d'une couche de fond (11) avec une multiplicité de cavités (10) disposées en un modèle ;
- agencement (34) de bandes de blister de réserve en différents postes de distribution d'un dispositif de remplissage, les bandes de blister de réserve présentant chacune en des positions prédéfinies des alvéoles (16) pour recevoir un nombre de médicaments différents sur chaque poste de distribution, et les alvéoles de blister de réserve (16) étant fermées (38) au moyen d'une feuille de scellement provisoire, appliquée de façon adhérente sur des profils de la bande de fond entre les alvéoles (16) ;
- décollement partiel (42) de la feuille de scellement provisoire de la bande de blister de réserve pour dégager un nombre prédéfini de médicaments sur le au moins un dispositif d'aspiration ou de prise (50) ;
- réception contrôlée (44) d'un nombre souhaité de médicaments à partir des positions prédéfinies sur chaque poste de distribution au moyen d'au moins un dispositif d'aspiration ou de prise (50) mobile et dépôt des médicaments dans des cavités prédéfinies (10), et
- application (46) d'une feuille de scellement (12) sur la couche de fond (11) pour la fermeture des cavités (10).

2. Procédé suivant la revendication 1, **caractérisé en ce que** la fabrication de la bande de blister de réserve comprend les étapes suivantes :
- formage (35) d'une bande de fond s'étendant largement dans la direction longitudinale et transversale, ce dont il résulte la multiplicité d'alvéoles de blister de réserve (16), et
- alimentation (36) des alvéoles de blister de réserve (16) par des médicaments.

3. Procédé suivant la revendication 2, **caractérisé par** l'étape supplémentaire de découper la bande de blister de réserve en sections de blister avant l'étape du décollement (42) de la feuille de scellement provisoire de la bande de blister de réserve, et d'agencer sur un rouleau les sections de blister de réserve au poste de distribution transversalement à la direction de transport de l'emballage pour plusieurs jours.

4. Procédé suivant l'une des revendications précédentes, **caractérisé en ce que** plusieurs dispositifs d'aspiration ou de prise (50) sont commandés individuellement sur au moins un poste de distribution.

5. Procédé suivant la revendication 4, **caractérisé en ce que** les dispositifs d'aspiration ou de prise (50) sont disposés en fonction du modèle de cavités (10) dans l'emballage pour plusieurs jours et sont déplacés parallèlement entre eux transversalement à la direction de transport de l'emballage pour plusieurs jours.

6. Procédé suivant l'une des revendications précédentes, **caractérisé en ce que** le au moins un dispositif d'aspiration ou de prise (50) est surveillé au moyen d'une caméra (54) ou par des capteurs.

7. Procédé suivant l'une des revendications précédentes, **caractérisé par** les étapes supplémentaires, consistant à pratiquer des perforations (2) dans la couche de fond (11) et dans la feuille de scellement (12) entre les cavités (10).
